# EUROPEAN PATENT APPLICATION

(11) **EP 2 609 880 A1**
(43) Date of publication of application: **03.07.2013**
(21) Application number: 12198755.6
(22) Date of filing: 20.12.2012
(51) Int. Cl.: A61B 17/34

(54) **Wound protector with reinforced ring**

(30) Priority: 29.12.2011 US 201161581203 P; 10.12.2012 US 201213709098
(71) Applicant: Covidien LP, Mansfield, MA 02048 (US)
(72) Inventor: Smith, Robert C., Middlefield, CT Connecticut 06455 (US)
(74) Representative: Soames, Candida Jane

(57) **Abstract**

A surgical sleeve is disclosed herein for insertion into an opening in tissue to minimize damage to the opening in tissue. The various embodiments of the surgical sleeve include a reinforcement member or a stiffening member for providing radial support to a proximal anchor member. In one embodiment, the surgical sleeve includes a proximal anchor member configured and dimensioned to contact an outer surface of the tissue to inhibit advancement of the surgical sleeve entirely through the opening, a distal anchor member configured and dimensioned to facilitate anchoring of the surgical sleeve within the opening in tissue, a sleeve member extending between the proximal anchor member and the distal anchor member, the sleeve member defining a passageway extending therethrough, and a reinforcement member positionable within the proximal anchor member to provide radial support to the proximal anchor member.

## Description

### CROSS REFERENCE TO RELATED APPLICATIONS

This application claims priority to, and the benefit of U.S. Provisional Patent Application Serial no. 61/581,203, filed December 29, 2011, the entire contents of which are incorporated herein by reference.

### BACKGROUND

### Technical Field

The present disclosure relates generally to surgical instruments for use with a surgical access portal. More particularly, the present disclosure relates to an adjustable sleeve for insertion into an opening in tissue to minimize damage to the opening in tissue.

### Description of Related Art

Increasingly, many surgical procedures are performed through small openings or natural openings in the skin. As compared to the larger openings typically required in traditional procedures, smaller openings result in less trauma to the patient. By reducing the trauma to the patient, the time required for recovery is also reduced. Generally, the surgical procedures that are performed through small openings in the skin are referred to as "endoscopic". If the procedure is performed on the patient's abdomen, the procedure is referred to as "laparoscopic". Throughout the present disclosure, the term "minimally invasive" is to be understood as encompassing both endoscopic and laparoscopic procedures.

During a typical minimally invasive procedure, surgical objects, such as surgical access devices (e.g., trocar and cannula assemblies) or endoscopes, are inserted into the patient's body through the opening in tissue. In general, prior to the introduction of the surgical object into the patient's body, insufflation gas is used to enlarge the area surrounding the target surgical site to create a larger, more accessible work area. Accordingly, the maintenance of a substantially fluid-tight seal is desirable so as to inhibit the escape of the insufflation gas and the deflation or collapse of the enlarged surgical site. In response to this, various access devices with sealing features are used during the course of minimally invasive procedures to provide an access for surgical objects to enter the patient's body. Each of these devices is configured for use through a single opening or a naturally occurring orifice (i.e. mouth, anus, or vagina) while allowing multiple instruments to be inserted through the device to access the working space beyond the device.

However, a continuing need exists for a way to minimize damage to the opening or incision in tissue.

### SUMMARY

A surgical sleeve is disclosed herein for insertion into an opening in tissue to minimize damage to the opening in tissue. The various embodiments of the surgical sleeve include a reinforcement member or a stiffening member for providing radial support to a proximal anchor member to prevent or limit the proximal anchor member from collapsing during use.

In one embodiment, the surgical sleeve includes a proximal anchor member configured and dimensioned to contact an outer surface of the tissue to inhibit advancement of the surgical sleeve entirely through the opening, a distal anchor member configured and dimensioned to facilitate anchoring of the surgical sleeve within the opening in tissue, a sleeve member extending between the proximal anchor member and the distal anchor member, the sleeve member defining a passageway extending therethrough, and a reinforcement member positionable within the proximal anchor member to provide radial support to the proximal anchor member.

The surgical sleeve is movable between an elongated configuration and a shortened configuration via manipulation of the proximal anchor member. The proximal anchor member has a non-circular cross-sectional configuration which maintains the shortened configuration of the outer member via engagement with the tissue. The reinforcement member may define a substantially circular shape and the proximal anchor member includes a slot therein for the reception of the reinforcement member. The proximal anchor member may receive the reinforcement member by, for example, a snap fit. The proximal anchor member may also include flexible arms extending from the slot where the flexible arms are adapted to flex outward to receive the reinforcement member within the slot.

The proximal anchor member may include a passage extending circumferentially therethrough for reception of the reinforcement member where the reinforcement member is insertable into the passage. The proximal anchor member and the passage may alternatively be formed about the reinforcement member.

The reinforcement member may be formed of a material which is more rigid than the proximal anchor member. The reinforcement member may formed of, for example, a rigid material or a resilient material.

The surgical sleeve may further include a stiffening member adapted for insertion into an opening of the proximal anchor member to provide radial support to the proximal anchor member where the stiffening member abuts an inner side of the proximal anchor member and is adapted to limit movement of the proximal anchor member in the radially inward direction.

In another embodiment, a surgical sleeve for insertion into an opening in tissue is disclosed including a proximal anchor member configured and dimensioned to contact an outer surface of the tissue to inhibit advancement of the surgical sleeve entirely through the opening and defining an opening therethrough, a distal anchor member configured and dimensioned to facilitate anchoring of the surgical sleeve within the opening in tissue, a sleeve member extending between the proximal anchor member and the distal anchor member and defining a passageway extending therethrough, and a stiffening member positionable within the opening of the proximal anchor member to provide radial support to the proximal anchor member. The surgical sleeve may be movable between an elongated configuration and a shortened configuration via manipulation of the proximal anchor member and the stiffening member is adapted to limit movement of the proximal anchor member in the radially inward direction.

A method of use for the surgical sleeve is also disclosed including inserting the surgical sleeve into an opening in tissue and inserting a reinforcement member into a proximal anchor member of the surgical sleeve to provide radial support to the proximal anchor member. The method may further include inserting a stiffening member into an opening of the proximal anchor member to provide radial support to the proximal anchor member.

### BRIEF DESCRIPTION OF THE DRAWINGS

The accompanying drawings, which are incorporated in and constitute a part of this specification, illustrate embodiments of the presently disclosed surgical sleeve, and together with a general description of the disclosed surgical sleeve given above, and the detailed description of the embodiments given below, serve to explain the principles of the disclosed surgical sleeve.

FIG. 1 is perspective view a surgical sleeve in accordance with the present disclosure;

FIG. 2A is a side cross-sectional view of the surgical sleeve of FIG. 1 inserted into an opening in tissue in accordance with an embodiment of the present disclosure;

FIG. 2B is a side cross-sectional view of the proximal anchor member of the surgical sleeve of FIG. 2A;

FIG. 2C is a side cross-sectional view of FIG. 2B with the reinforcement member inserted into the slot;

FIG. 2D is a top cross-sectional view of the proximal anchor member of the surgical sleeve of FIG. 2A;

FIG. 2E is a top view of an embodiment of the reinforcement member prior to insertion into the proximal anchor member of FIG. 2A shown in a substantially linear state;

FIG. 2F is a top view of an embodiment of the reinforcement member of FIG. 2E in a curved state;

FIG. 2G is an alternate embodiment of the reinforcement member of FIG. 2E including multiple sections;

FIG. 3A is a side cross-sectional view of the surgical sleeve of FIG. 1 inserted into an opening in tissue in accordance with another embodiment of the present disclosure;

FIG. 3B is a side cross-sectional view of the proximal anchor member of the surgical sleeve of FIG. 3A;

FIG. 3C is a side cross-sectional view of FIG. 3B with the reinforcement member inserted into the passage;

FIG. 3D is a top cross-sectional view of the proximal anchor member of the surgical sleeve of FIG. 3A prior to insertion of the reinforcement member;

FIG. 3E is a top cross-sectional view of the proximal anchor member of the surgical sleeve of FIG. 3D after insertion of the reinforcement member;

FIG. 3F is a top cross-sectional view of the proximal anchor member of the surgical sleeve of FIG. 3A with the proximal anchor member formed about the reinforcement member;

FIG. 4 is a side cross-sectional view of the surgical sleeve of FIG. 1 inserted into an opening in tissue in accordance with another embodiment of the present disclosure showing a stiffening member;

FIG. 5 is a side cross-sectional view of the surgical sleeve of FIG. 4 with the stiffening member inserted into the opening of the proximal anchor member; and

FIG. 6 is a side cross-sectional view of the surgical sleeve of FIG. 5 including a valve assembly.

### DETAILED DESCRIPTION

Various embodiments of the presently disclosed surgical sleeve, and methods of using the same, will now be described in detail with reference to the drawings wherein like references numerals identify similar or identical elements. In the drawings, and in the following description, the term "proximal" should be understood as referring to the end of the pertinent structure that is closer to the clinician during proper use, while the term "distal" should be understood as referring to the end that is farther from the clinician, as is traditional and conventional in the art. Additionally, use of the term "opening" herein below should be understood to encompass any opening in a patient's tissue, including natural openings (e.g. anus, vagina and mouth) and surgically formed openings.

Disclosed herein is a surgical sleeve for minimizing damage to an incision or opening in tissue. More specifically a surgical sleeve including a reinforcement member positionable within a proximal anchor member is disclosed which is insertable into a single opening or natural orifice in a body and adapted to minimize or prevent additional damage to the opening or natural orifice after insertion by surgical instruments or objects inserted therethrough.

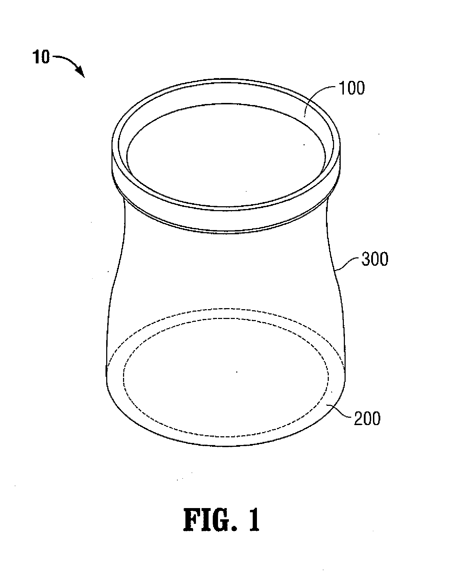

FIG. 1 illustrates one embodiment of the presently disclosed surgical sleeve, which is identified by the reference character 10, in use during the course of a minimally invasive surgical procedure. Although described in the context of a laparoscopic surgical procedure herein below, it should be understood that the surgical sleeve 10 may be utilized during any minimally invasive surgical procedure.

The surgical sleeve 10 includes a proximal anchor member 100, a distal anchor member 200, and a sleeve member 300. In the absence of an indication to the contrary, it should be understood that the various components of surgical sleeve 10 are formed from a biocompatible material.

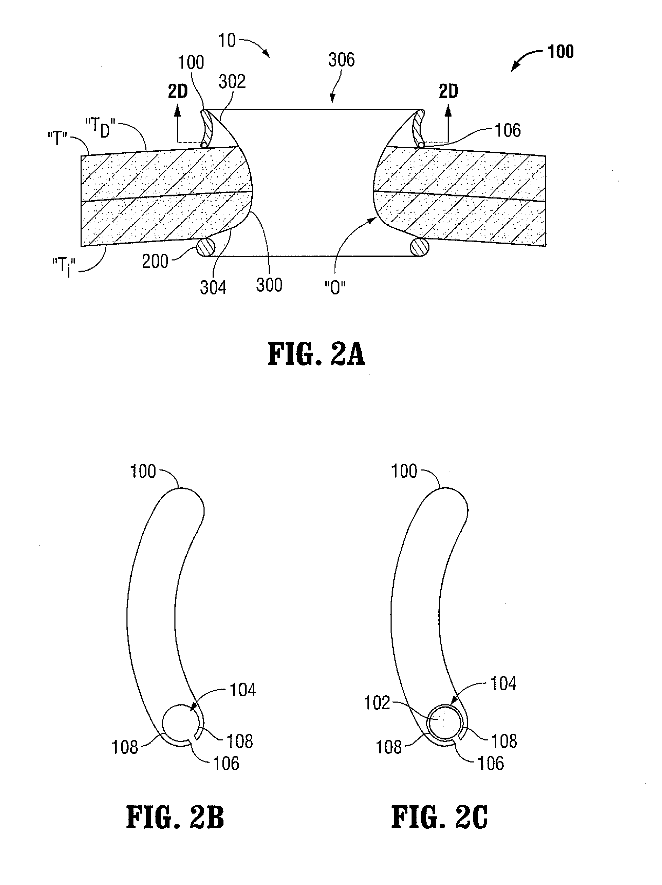

With reference now to FIGS. 2A-2G, the surgical sleeve 10 is configured and dimensioned for insertion into an opening "0" formed in a patient's tissue "T".

The proximal anchor member 100 is configured and dimensioned for engagement with an outer surface of the tissue "Tₒ" to inhibit advancement of the proximal anchor member 100 through the opening "O", and facilitate securement of the surgical sleeve 10 relative to the tissue "T". Although illustrated as substantially annular in configuration, it should be understood that the proximal anchor member 100 may assume any suitable geometrical configuration, such as, for example, an elliptical or rectangular configuration.

In one embodiment of surgical sleeve 10, sleeve member 300 is movable between an elongated configuration and a shortened configuration via manipulation of the proximal anchor member 100. The proximal anchor member 100 may have a non-circular transverse cross-sectional configuration adapted for maintaining the shortened configuration of the sleeve member 300 by engagement with an outer surface of tissue "Tₒ". For example, the transverse cross-sectional configuration may be kidney shaped, crescent shaped, or other shapes suitable for engaging the outer surface of tissue "Tₒ".

With reference to FIGS. 2B-2G, the proximal anchor member 100 may include a reinforcement member 102 which is positionable within the proximal anchor member 100 to support or reinforce proximal anchor member 100 and to prevent or limit proximal anchor member 100 from unintentionally collapsing into the opening "O" in tissue "T" during the surgical operation. Proximal anchor member 100 may include a slot 104 at an end 106 for receiving reinforcement member 102 and reinforcement member 102 may be secured within slot 104 by a snap fit, friction fit, or other suitable mechanism for securing reinforcement member 102 within slot 104. Slot 104 may alternatively be disposed in any other part of proximal anchor member 100 as desired. Reinforcement member 102 may also be secured within slot 104 through the use of a locking mechanism such as, for example, a latch.

Slot 104 may include arm members 108 (FIGS. 2B and 2C) formed of a resilient material such as, for example, a flexible polymeric material or a shape memory material having an initial configuration and being adapted to spread apart to an expanded configuration to receive reinforcement member 102 as reinforcement member 102 is inserted into slot 104. Arm members 108 may be biased towards the initial configuration and may transition to the expanded configuration when reinforcement member 102 is inserted into slot 104 due to the force being applied by reinforcement member 102 on arm members 108. Once reinforcement member 102 is positioned within slot 104, arm members 108 return to the initial configuration due to the biasing forces and secure reinforcement member 102 within slot 104. Proximal anchor member 100 may be rotatable about or around reinforcement member 102 when reinforcement member 102 is inserted into slot 104 to transition the sleeve member 300 between the elongated configuration and the shortened configuration. Alternatively the insertion of reinforcement member 102 into slot 104 may prevent or limit proximal anchor member 100 from being rotated or manipulated such that sleeve member 300 is no longer transitionable between the elongated configuration and the shortened configuration.

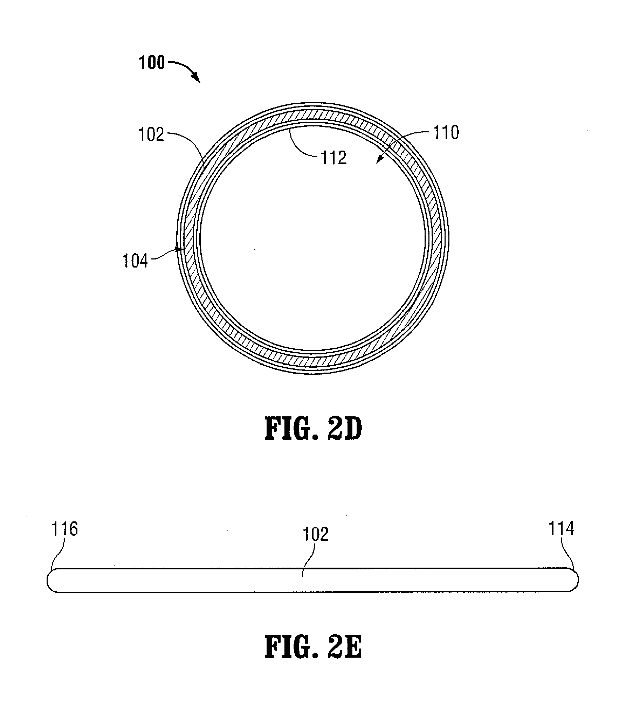

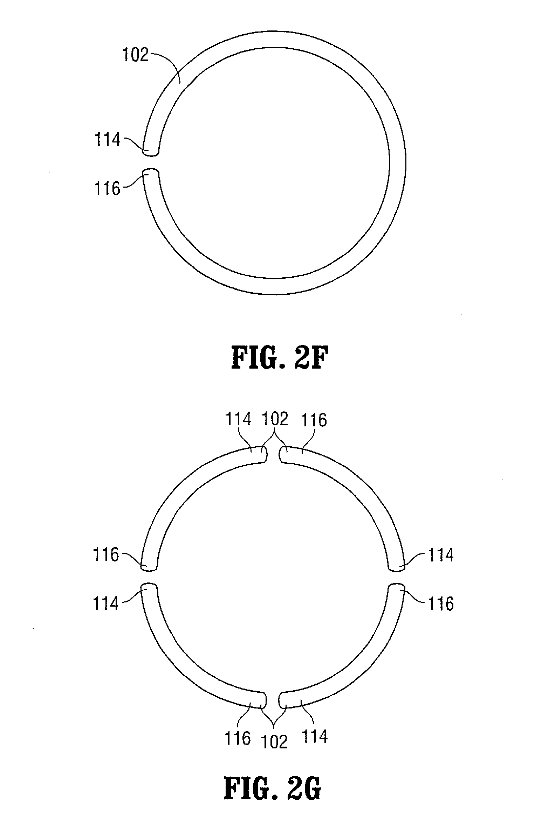

Reinforcement member 102 is configured and dimensioned to extend at least partially around the circumference of proximal anchor member 100 and may define a transverse cross-sectional profile which is circular, oval, square, triangle, rectangle, or any other shape suitable for the purpose of supporting or reinforcing proximal anchor member 100. Reinforcement member 102 may be formed of a single section (FIGS. 2D-2F) or may include multiple sub-sections (FIG. 2G) each of which is independently insertable into a portion of slot 104. In one embodiment, reinforcement member 102 may be initially biased in a substantially linear state (FIG. 2E) having a first end 114 and a second end 116 and may be bendable or deformable into a substantially arcuate or curved state (FIG. 2F) for insertion into slot 104. One of first and second ends 114 and 116 may alternatively be inserted into slot 104 and reinforcement member 102 may be threaded into slot 104. Once inserted into slot 104, reinforcement member 102 provides proximal anchor member 100 with a radially outward biasing force against slot 104 to support proximal anchor member 100 and prevent or limit the unintentional collapse of proximal anchor member 100 into the opening "O" in tissue "T" during a surgical procedure. Reinforcement member 102 may be formed of a resilient material, such as, e.g., a flexible polymeric material, or a shape memory material which is capable of deforming and subsequently returning to its original shape or may be formed of a substantially more rigid material, such as, e.g., an acrylonitrile butadiene styrene ("ABS") polymer, where reinforcement member 102 would be pre-formed for insertion into slot 104 of proximal anchor member 100.

In one embodiment of the surgical sleeve 10, the proximal anchor 110 may be formed from a resilient material that allows for repositioning between an expanded configuration and a compressed configuration. For example, the proximal anchor may be formed from a flexible polymeric material. Alternatively, however, the proximal anchor member 100 may formed from a substantially more rigid material, e.g., an ABS polymer.

Referring again to FIG. 2A, the distal anchor member 200 is configured and dimensioned for engagement with an internal surface of the tissue "T_{I}" to inhibit unintentional withdrawal of the surgical sleeve 10 from the opening "O", and facilitate securement of the surgical sleeve 10 relative to the tissue "T." Although illustrated as substantially annular in configuration, it should be understood that the distal anchor member 200 may assume any suitable geometrical configuration, such as, for example, an elliptical or rectangular configuration.

In one embodiment, for example, it is envisioned that the distal anchor member 200 may be formed from an at least partially resilient material, such as a flexible polymeric material. The resilient material comprising the distal anchor member 200 allows for repositioning of the distal anchor member 200 between an expanded configuration and a compressed configuration that facilitates passage of the distal anchor member 200 through the opening "O" in the tissue "T." The respective proximal and distal anchor members 100, 200 may also be formed from the same material, or from different materials. For example, it is envisioned that the respective proximal and distal anchor members 100, 200 may each be formed from a resilient, flexible material, or that the material comprising the proximal anchor member 100 may be appreciably more rigid than the material comprising the distal anchor member 200. It is also envisioned that distal anchor member 200 may be more rigid than proximal anchor member 100 and reinforcement member 102 may have the same rigidity or greater rigidity than distal anchor member 200 such that upon insertion of reinforcement member 102 into slot 104, proximal anchor member 100 becomes the same or more rigid than distal anchor member 200.

With continued reference to FIG. 2A, the sleeve member 300 extends between, and connects, the respective proximal and distal anchor members 100, 200. Specifically, the sleeve member 300 includes a proximal end 302 that is connected to the proximal anchor member 100, and a distal end 304 that is connected to the distal anchor member 200. The sleeve member 300 may be connected to the respective proximal and distal anchor members 100, 200 though any suitable means, e.g., via heat sealing, mechanical connection, or through the use of an adhesive. Alternatively, it is envisioned that the sleeve member 300 may be integrally formed with either, or both, of the respective proximal and distal anchor members 100, 200.

The sleeve member 300 provides a passageway 306 extending through the surgical sleeve 10 that is configured and dimensioned to receive a surgical access device or surgical instrument therethrough (not shown). Although illustrated as being substantially cylindrical in configuration in FIGS. 1 and 2A, it is envisioned that the sleeve member 300 may assume any suitable geometrical configuration. For example, it is envisioned that the sleeve member 300 may have an hourglass configuration, or that the sleeve member 300 may be elliptical in configuration.

In one embodiment of surgical sleeve 10, it is envisioned that the material comprising the sleeve member 300 may be impermeable to fluids and/or bacteria, whereby the sleeve member 300 forms a substantially fluid-tight seal with the opening "O" in the tissue "T." Alternatively, however, it is envisioned that the sleeve member 300 may be adapted to facilitate the communication of fluid therethrough, either by the material of construction, or by the inclusion of one or more openings therein.

Additionally, although not described as such herein below, it is envisioned that either or both of the respective proximal and distal anchor members 100, 200 of the surgical sleeve 10 may be adapted for inflation via the inclusion of an internal space that is adapted to receive a fluid communicated from an external source, e.g., through an inflation port.

With reference now to FIGS. 2A-2B, the use and function of the surgical sleeve 10 will be discussed. During the course of a typical minimally invasive procedure, the internal worksite is insufflated with a suitable biocompatible gas, e.g., CO₂, such that the internal walls of the worksite are raised and lifted away from the organs and tissue housed therein. The insufflation may be performed with an insufflation needle or similar device, as is conventional in the art.

The distal anchor member 200 of the surgical sleeve 10 is prepared for insertion into the opening "O" in tissue "T" by transitioning distal anchor member 200 from the expanded configuration to the compressed configuration. Once in the compressed configuration, distal anchor member 200 is inserted into opening "O" formed in the tissue "T".

Once inserted into opening "O", distal anchor member 200 is allowed to return to the expanded configuration where distal anchor member 200 is expanded beneath the tissue "T" to engage the inner surface "Tᵢ". The surgical sleeve 10 is arranged according to the illustration provided in FIG. 2A, whereby the distal anchor member 200 is positioned beneath (distally of) the tissue "T," and the proximal anchor member 100 is positioned above (proximally of) the tissue "T," so as to facilitate anchoring of the surgical sleeve 10 within the opening "O." Proximal anchor member 100 is then manipulated to move or transition the sleeve member 300 between the elongated configuration and the shortened configuration as necessary depending on the size of the opening "O" in tissue "T". For example, in a large opening "O" proximal anchor member 100 may be manipulated toward the elongated configuration to increase the length of sleeve member 300 while in a smaller opening "O" proximal anchor member 100 may be manipulated toward the shortened configuration to decrease the length of sleeve member 300. Proximal anchor member 300 may be rotated about itself to increase or decrease the length of sleeve member 300 where sleeve member 300 may be wrapped around proximal anchor member 300. In this way the surgical sleeve 10 may be adjusted to fit any size of opening "O" in a patient's tissue "T".

Reinforcement member 102 may be inserted into slot 104 (FIGS. 2C, 2D) of proximal anchor member 100 before or after proximal anchor member 100 has been manipulated to provide additional support for proximal anchor member 100. Proximal anchor member 100 may be rotated about reinforcement member to transition sleeve member 300 between the elongated and shortened configurations.

Once reinforcement member 102 has been inserted into slot 104, surgical objects or the surgical access devices (not shown) may be inserted through passageway 306 of sleeve member 300 to perform a surgical operation. After completion of the surgical operation and removal of the surgical objects from passageway 306, surgical sleeve 10 may be removed from the opening "O" in tissue "T".

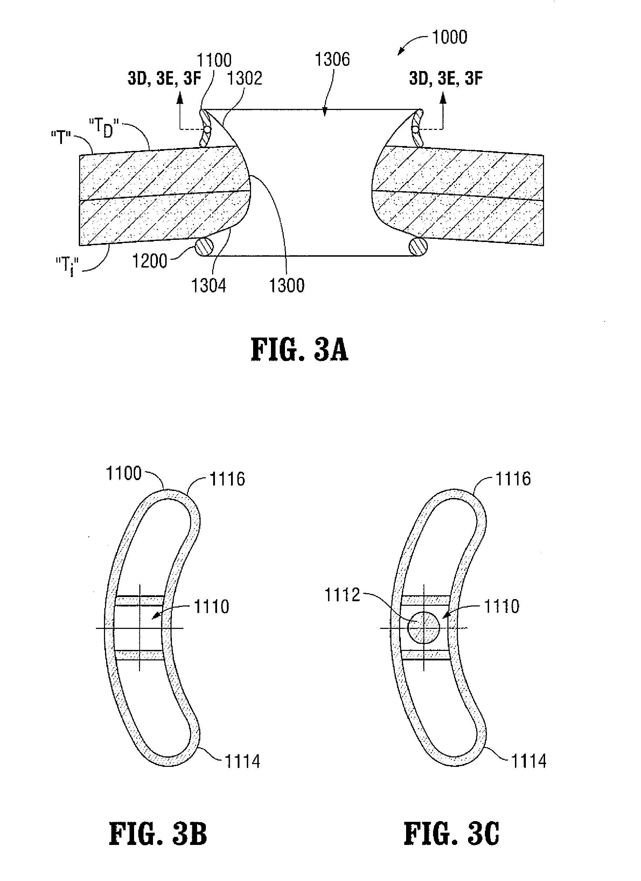

In an alternate embodiment, illustrated in FIGS. 3A-3F, a surgical sleeve 1000 is disclosed which is similar to the above disclosed surgical sleeve 10. For brevity only the differences between surgical sleeve 10 and surgical sleeve 1000 will be discussed in further detail below where similar reference numerals identify similar elements. As discussed above with regard to surgical sleeve 10, surgical sleeve 1000 includes a proximal anchor member 1100, a distal anchor member 1200 and a sleeve member 1300. Sleeve member 1300 includes a proximal end 1302, a distal end 1304 and a passageway 1306 extending therethrough as discussed above with regard to sleeve member 300.

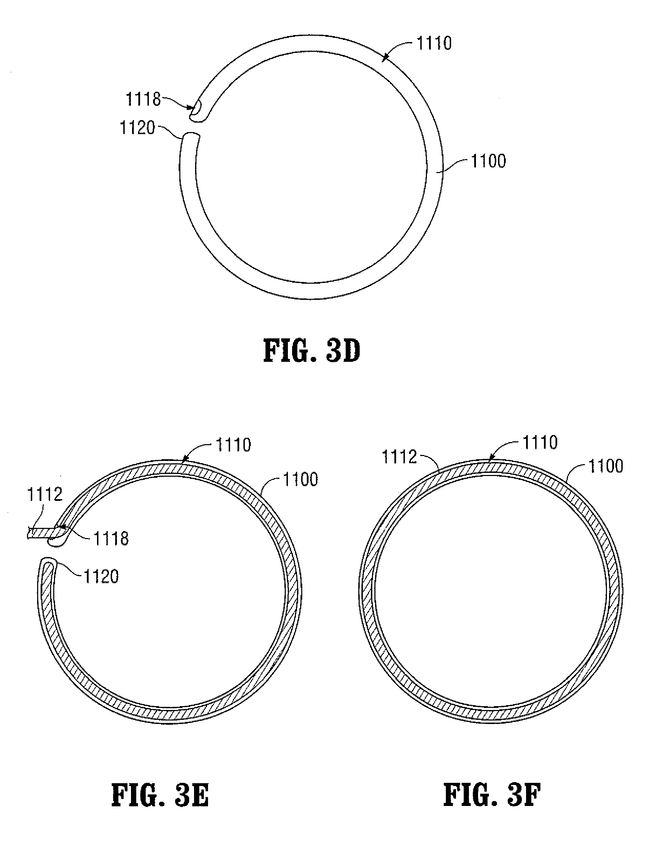

In this embodiment, with reference to FIGS. 3A-3D, proximal anchor member 1100 includes a passage or channel 1110 extending circumferentially therethrough and adapted to receive a reinforcement member 1112 therethrough. Passage 1110 may be disposed within proximal anchor member 1100 near one of first and second ends 1114 and 1116 or may be disposed at a position between first and second ends 1114 and 1116. For example, passage 1110 may be disposed halfway between first and second ends 1114 and 1116. As seen in FIGS. 3D and 3E, passage 1110 may include an opening or inlet 1118 and may extend to a closed end 1120. Closed end 1120 may be disposed anywhere along the circumference of proximal anchor member 1100 and may, for example, abut or be proximal to opening or inlet 1118. Alternatively, passage 1110 may be entirely enclosed where proximal anchor member 1100 is formed or extruded about passage 1110.

Reinforcement member 1112 may be removably insertable into passage 1110 through an opening or inlet 1118 or may be positioned within passage 1110 prior to the forming of proximal anchor member 1100 about passage 1110 as discussed above where, for example, reinforcement member 1112 is fully enclosed within passage 1110 (FIG. 3F). Reinforcement member 1112 may, for example, be in the shape of a hoop or a ring or may be a resilient, flexible or semi-rigid rod. Reinforcement member 1112 may define a transverse cross-sectional profile which is circular, oval, square, triangle, rectangle, or almost any other shape suitable for the purpose of supporting or reinforcing proximal anchor member 1100. Reinforcement member 1112 is adapted extend through passage 1110 around the circumference of proximal anchor member 1100 to support or reinforce proximal anchor member 1100 and to prevent or limit proximal anchor member 1100 from unintentionally collapsing into the opening "O" in tissue "T" during the surgical operation. Reinforcement member 1112 may, for example, extend into inlet 1118 and through passage 1110 to closed end 1120 (FIG. 3E).

Passage 1110 may be formed such that reinforcement member 1112 is slidable or moveable with respect to passage 1110 and proximal anchor member 1100 may be rotatable or manipulatable about reinforcement member 1112 to transition sleeve member 1300 is between the elongated configuration and the shortened configuration as discussed above with regard to surgical sleeve 10.

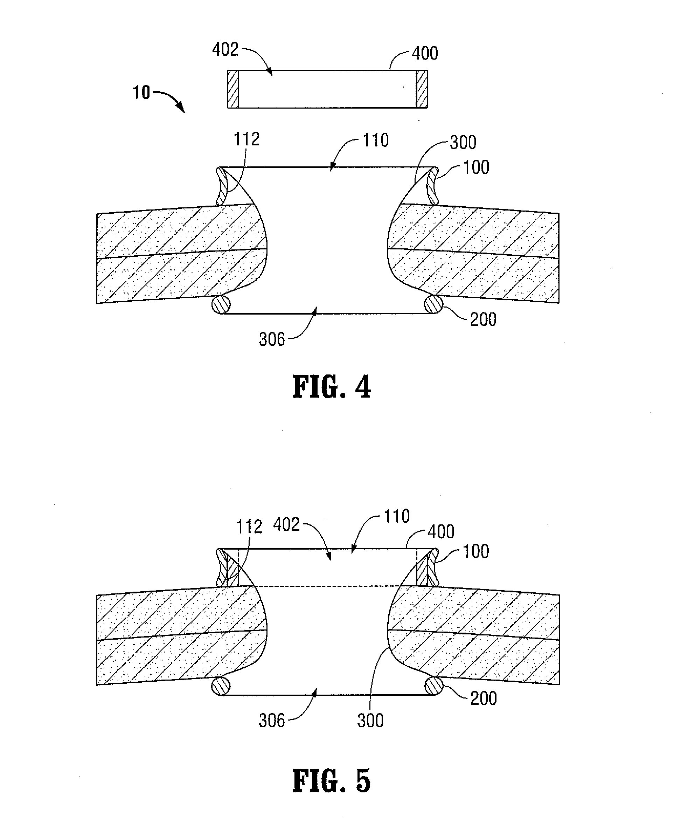

In another embodiment, as illustrated in FIGS. 4 and 5, a stiffening member 400 is disclosed for insertion into an opening 110 defined by proximal anchor member 100. Stiffening member 400 includes a lumen 402 extending therethrough and is configured and dimensioned to be positioned adjacent to or abutting an inner side 112 of proximal anchor member 100 when inserted into opening 110 (FIG. 5) to prevent or limit proximal anchor member 100 from rolling or collapsing into the opening "O" in tissue "T". Stiffening member 400 may be formed of a semi-rigid material or a rigid material such as, for example, an acrylonitrile butadiene styrene ("ABS") polymer or other materials suitable for preventing proximal anchor member 100 from collapsing into the opening "O" in tissue "T" during a surgical operation.

Stiffening member 400 may alternatively be transitionable between a collapsed state and an expanded state where, for example stiffening member 400 is collapsed prior to insertion into the opening 110 of proximal anchor member 100. After insertion into opening 110 stiffening member 400 may transition to the expanded state. For example, stiffening member 400 may be formed of a resilient material or a shape memory material and may be biased in the expanded state. The expanded state may have a diameter which is greater than the diameter of the inner side 112 of proximal anchor member 100 such that when stiffening member 400 is transitioned to the expanded state after insertion the stiffening member 400 abuts and presses against inner side 112 of proximal anchor member 100 to prevent proximal anchor member 100 from collapsing. The diameter of stiffening member 400 in the expanded state may alternatively be equal to or less than the diameter of the inner side 112 of proximal anchor member 100. Stiffening member 400 may also be inflatable and may include a reservoir for receiving a fluid from a fluid source, where the stiffening member 400 is transitionable between the collapsed state to the expanded state by the introduction of a fluid from the fluid source into the reservoir or removal of a fluid from the reservoir into the fluid source. Stiffening member 400 may also be used with surgical sleeve 1000.

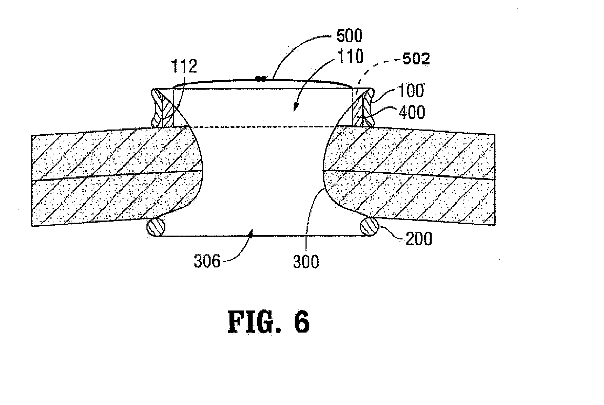

Referring now to FIG. 6, stiffening member 400 may form a substantially fluid tight seal with inner side 112 of proximal anchor member 100 when inserted into opening 110 and may include a valve assembly 500, e.g., an iris seal valve or other similar valves. For example, stiffening member 400 may define a distal portion or ring 502 of the valve assembly 500 and may be configured to fluidly seal and secure valve assembly 500 to proximal anchor member 100. Valve assembly 500 is configured to allow a surgical object to be inserted through opening 110 in a substantially fluid sealed manner. In one embodiment, for example stiffening member 400 may engage against a portion of sleeve 300 adjacent proximal anchor member 100 when inserted into opening 110 to form a fluid tight seal with sleeve 300. In another embodiment, for example, the stiffening member 400 may fluidly seal against the inner side 112 of opening 110 when transitioned to the expanded state. In another embodiment, for example, the proximal anchor member 100 may contract or collapse against stiffening member 400 to form the fluid tight seal. The use of valve assembly 500 assists in maintaining an insufflated environment within the body cavity during a surgical procedure.

Although the illustrative embodiments of the present disclosure have been described herein with reference to the accompanying drawings, the above description, disclosure, and figures should not be construed as limiting, but merely as exemplifications of particular embodiments. It is to be understood, therefore, that the disclosure is not limited to the precise embodiments described herein, and that various other changes and modifications may be effected by one skilled in the art without departing from the scope or spirit of the present disclosure.

The invention may be described by reference to the following numbered paragraphs:-
1. A surgical sleeve for insertion into an opening in tissue comprising:
   a proximal anchor member configured and dimensioned to contact an outer surface of the tissue to inhibit advancement of the surgical sleeve entirely through the opening, the proximal anchor member defining an opening therethrough;
   a distal anchor member configured and dimensioned to facilitate anchoring of the surgical sleeve within the opening in tissue;
   a sleeve member extending between the proximal anchor member and the distal anchor member, the sleeve member defining a passageway extending therethrough; and
   a stiffening member positionable within the opening of the proximal anchor member to provide radial support to the proximal anchor member.
2. A surgical sleeve according to paragraph 1, wherein the surgical sleeve is movable between an elongated configuration and a shortened configuration via manipulation of the proximal anchor member.
3. A surgical sleeve according to paragraph 2, wherein the proximal anchor member has a non-circular cross-sectional configuration which maintains the shortened configuration of the outer member via engagement with the tissue.
4. A surgical sleeve according to paragraph 1, wherein the stiffening member is configured to limit movement of the proximal anchor member in a radially inward direction.
5. A surgical sleeve according to paragraph 4, wherein the stiffening member abuts an inner side of the proximal anchor member to limit movement of the proximal anchor member in the radially inward direction.
6. A surgical sleeve according to paragraph 1, wherein the stiffening member is formed of a rigid material.
7. A surgical sleeve according to paragraph 1, wherein the stiffening member is transitionable between a collapsed state and an expanded state.
8. A surgical sleeve according to paragraph 7, wherein the stiffening member is formed of a resilient material
9. A surgical sleeve according to paragraph 7, wherein the stiffening member is formed of a shape memory material.
10. A surgical sleeve according to paragraph 7, wherein the stiffening member is biased in the expanded state.
11. A surgical sleeve according to paragraph 7, wherein the stiffening member defines a diameter which is greater than the diameter of an inner side of the proximal anchor member when the stiffening member is in the expanded state.
12. A surgical sleeve according to paragraph 1, wherein the stiffening member is configured to form a substantially fluid tight seal with the proximal anchor member.
13. A surgical sleeve according to paragraph 1, wherein the stiffening member further includes a valve assembly attached thereto, the valve assembly configured to fluidly seal the opening in the proximal anchor member.
14. A method of use for a surgical sleeve comprising:
   inserting the surgical sleeve into an opening in tissue;
   positioning a distal anchor member of the surgical sleeve against an inner surface of the opening to inhibit withdrawal of the surgical sleeve from the opening in tissue;
   positioning a proximal anchor member of the surgical sleeve against an outer surface of the opening, the proximal anchor member defining a passageway extending therethrough;
   inserting a stiffening member into the passageway of the proximal anchor member to provide radial support to the proximal anchor member.
15. The method according to paragraph 14, wherein the stiffening member is configured to limit movement of the proximal anchor member in a radially inward direction.
16. The method according to paragraph 14, further including the step of manipulating the proximal anchor member to move the surgical sleeve between an elongated configuration and a shortened configuration.
17. The method according to paragraph 16, wherein the proximal anchor member is moved toward the shortened configuration after the stiffening member is inserted therein, the proximal anchor member engaging the stiffening member to form a substantially fluid tight seal therewith.
18. The method according to paragraph 14, wherein the step of inserting the stiffening member further includes transitioning the stiffening member from a collapsed state to an expanded state to engage an inner side of the proximal anchor member.
19. The method according to paragraph 18, wherein the stiffening member forms a substantially fluid tight seal with the proximal anchor member when engaged with the inner side of the proximal anchor member.
20. The method according to paragraph 14, wherein the stiffening member includes a valve assembly attached thereto, the valve assembly substantially fluid sealing the passageway of the proximal anchor member when the stiffening member is inserted therein.

## Claims

1. A surgical sleeve for insertion into an opening in tissue comprising:
a proximal anchor member configured and dimensioned to contact an outer surface of the tissue to inhibit advancement of the surgical sleeve entirely through the opening, the proximal anchor member defining an opening therethrough;
a distal anchor member configured and dimensioned to facilitate anchoring of the surgical sleeve within the opening in tissue;
a sleeve member extending between the proximal anchor member and the distal anchor member, the sleeve member defining a passageway extending therethrough; and
a stiffening member positionable within the opening of the proximal anchor member to provide radial support to the proximal anchor member.

2. A surgical sleeve according to claim 1, wherein the surgical sleeve is movable between an elongated configuration and a shortened configuration via manipulation of the proximal anchor member; preferably wherein the proximal anchor member has a non-circular cross-sectional configuration which maintains the shortened configuration of the outer member via engagement with the tissue.

3. A surgical sleeve according to claim 1 or claim 2, wherein the stiffening member is configured to limit movement of the proximal anchor member in a radially inward direction; preferably wherein the stiffening member abuts an inner side of the proximal anchor member to limit movement of the proximal anchor member in the radially inward direction.

4. A surgical sleeve according to any preceding claim, wherein the stiffening member is formed of a rigid material.

5. A surgical sleeve according to any of claims 1 to 3, wherein the stiffening member is transitionable between a collapsed state and an expanded state.

6. A surgical sleeve according to claim 5, wherein the stiffening member is formed of a resilient material; or wherein the stiffening member is formed of a shape memory material.

7. A surgical sleeve according to claim 5, wherein the stiffening member is biased in the expanded state.

8. A surgical sleeve according to claim 5 or claim 7, wherein the stiffening member defines a diameter which is greater than the diameter of an inner side of the proximal anchor member when the stiffening member is in the expanded state.

9. A surgical sleeve according to any preceding claim, wherein the stiffening member is configured to form a substantially fluid tight seal with the proximal anchor member.

10. A surgical sleeve according to any preceding claim, wherein the stiffening member further includes a valve assembly attached thereto, the valve assembly configured to fluidly seal the opening in the proximal anchor member.

11. A method of use for a surgical sleeve comprising:
inserting the surgical sleeve into an opening in tissue;
positioning a distal anchor member of the surgical sleeve against an inner surface of the opening to inhibit withdrawal of the surgical sleeve from the opening in tissue;
positioning a proximal anchor member of the surgical sleeve against an outer surface of the opening, the proximal anchor member defining a passageway extending therethrough;
inserting a stiffening member into the passageway of the proximal anchor member to provide radial support to the proximal anchor member.

12. The method according to claim 11, wherein the stiffening member is configured to limit movement of the proximal anchor member in a radially inward direction.

13. The method according to claim 11 or claim 12, further including the step of manipulating the proximal anchor member to move the surgical sleeve between an elongated configuration and a shortened configuration.

14. The method according to claim 13, wherein the proximal anchor member is moved toward the shortened configuration after the stiffening member is inserted therein, the proximal anchor member engaging the stiffening member to form a substantially fluid tight seal therewith.

15. The method according to any of claims 11 to 14, wherein the step of inserting the stiffening member further includes transitioning the stiffening member from a collapsed state to an expanded state to engage an inner side of the proximal anchor member.
